# DEMANDE DE BREVET EUROPEEN

(11) **EP 0 611 232 A1**
(43) Date de publication de la demande: **17.08.1994**
(21) Numéro de dépôt: 94420040.1
(22) Date de dépôt: 07.02.1994
(51) Int. Cl.: C07C 235/42, C07C 235/82, C07C 235/60, C07D 295/192, C07C 231/14

(54) **Procédé pour la préparatiuon de dérivés phénylbenzamides**

(30) Priorité: 08.02.1993 FR 9301558
(71) Demandeur: RHONE-POULENC AGROCHIMIE, 69009 Lyon (FR)
(72) Inventeur: Schmitz, Christian, Pommiers, F-69480 Anse (FR)
(74) Mandataire: Chrétien, François

(57) **Abrégé**

1) Procédé pour le préparation de dérivés phénylbenzamides dérivés phénylbenzamides de formule A, 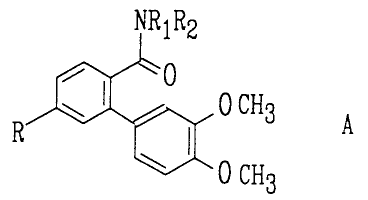 avec R: alkyle(C1-C2), substitué par 2 à 5 F,
   R1 et R2, identiques ou différents sont méthyle ou éthyle ou peuvent former ensemble un groupe morpholino,
2) on effectue une condensation d'une haloénone I avec un acétoamide II, selon le schéma: 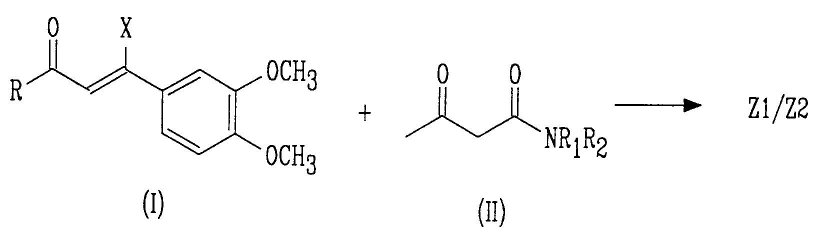 en présence d'un solvant organique et d'un à deux équivalents de base, et qu'on soumet le produit résultant à une transformation en deux étapes dont une de réduction.
3) les composés A sont des fongicides agricoles.

## Description

La présente invention a pour objet un nouveau procédé pour le préparation de dérivés phénylbenzamides .

Il est connu, notamment d'après la demande européenne 360 701, que certains de ces composés présentent d'intéressantes propriétés fongicides. Cette demande décrit, en plus des propriétés de ces produits, un procédé pour leur préparation. Un tel procédé donne des rendements satisfaisants au stade du laboratoire. Cependant ses performances sont insuffisantes pour une production industrielle. De plus certaines étapes, comme le couplage biarylique, qui utilisent des catalyseurs onéreux comme ceux à base de métaux précieux ou des organométalliques, sont coûteuses. D'autres étapes comme les diazotations sont dangereuses. Il est donc nécessaire de trouver une voie nouvelle plus propice à la fabrication à grande échelle.

La présente invention concerne un procédé ne présentant pas les inconvénients ci-dessus. Elle a plus particulièrement pour objet un procédé de préparation de composés de formule A, 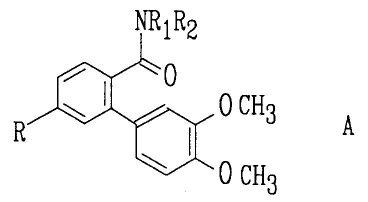
dans laquelle R est un alkyle de 1 à 2 atomes de carbone, substitué par 2 à 5 atomes de fluor, R ne pouvant pas être un trifluorométhyle, R1 et R2, identiques ou différents sont chacun un méthyle ou un éthyle ou peuvent former ensemble un groupe morpholino, caractérisé en ce qu'on effectue une condensation d'une haloénone de formule I, dans laquelle R a la même signification que ci-dessus, avec un acétoamide de formule II, dans laquelle R1 et R2 ont les même significations que ci-dessus selon le schéma: 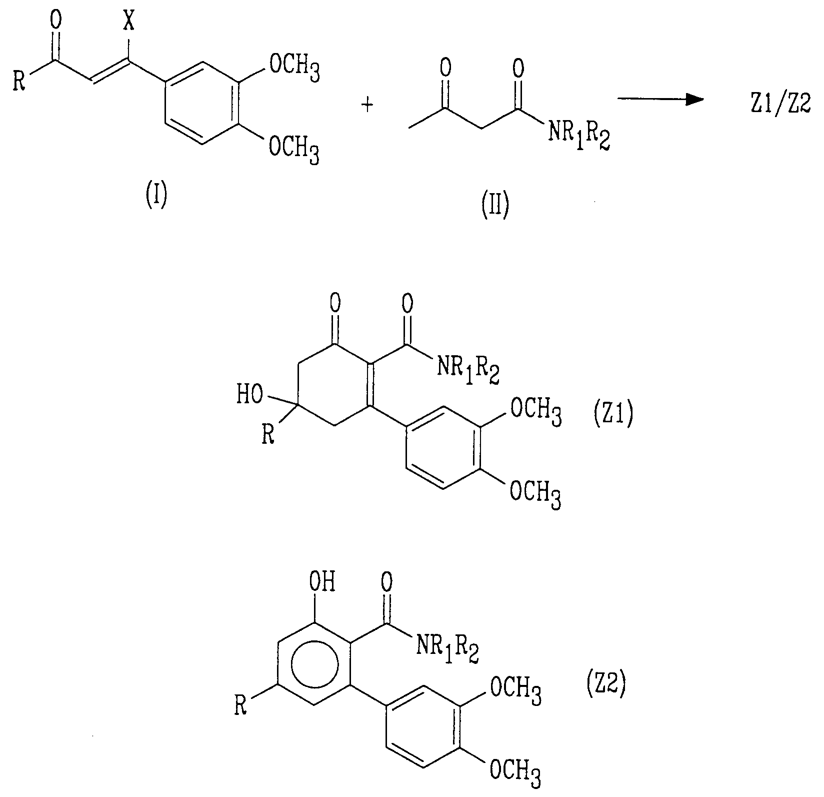
en présence d'un solvant organique et d'un à deux équivalents de base, et qu'on soumet le produit résultant à une transformation en deux étapes dont une de réduction.

Le rapport molaire Z1/Z2 dépend de la quantité de base mise en oeuvre, Z1 étant obtenu seul pour une quantité d'un équivalent de base, Z2 étant obtenu seul pour une quantité de deux équivalents de base, un mélange Z1/Z2 étant obtenu pour une quantité de base intermédiaire entre un et deux équivalents, le rapport variant en fonction de la durée de réaction et de la température.

Parmi les très nombreux couples "base - solvant" possibles pour réaliser cette condensation, on utilise avantageusement un alcool aliphatique tel que l'éthanol et une base minérale, en quantité stoéchiométrique, dérivée d'un métal alcalin ou alcalino-terreux telle que la baryte. Pratiquement, lorsque l'haloénone est une chloroénone, celle-ci est mise en solution avec l'acétoacétamide (1-1,1 équivalent) dans l'éthanol absolu à une température comprise entre 10°C et 30°C puis traitée par la base (1-1,1 équivalent) éventuellement ajoutée en plusieurs fois. Lorsque toute la chloroénone a réagi, on chauffe progressivement le mélange réactionnel jusqu'au reflux pour terminer la réaction (6 à 48 heures). Le mélange réactionnel refroidi est filtré puis concentré; le résidu est cristallisé dans un solvant tel que l'acétate d'éthyle.

Les acétoacétamides II sont des produits connus et le plus souvent dans le commerce. Les acétoacétamides II sont des produits connus et le plus souvent dans le commerce. Ils peuvent être préparés selon l'un des deux voies décrites dans la littérature: selon la première on fait agir du dicétène sur une amine HNR1R2(Chemical reviews 1986 vol 86, p 241); selon la seconde, on effectue une transamidification d'un acétoacétate d'alkyle commercial, de préférence l'acétate de t-butyle plus réactif que l'acétoacétate de méthyle ou d'éthyle(Journal of Organic Chemistry), par une amine HNR1R2.

Les composés haloénones I sont des produits nouveaux qu'on peut obtenir, de manière en soi connue, par halogénation des diones correspondantes, de formule V, selon le schéma: 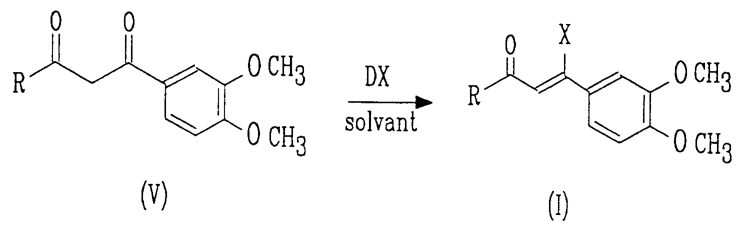
dans lequel R a la même signification que dans la formule du composé A et DX est un agent halogénant dont la partie labile est X.
La réaction s'effectue en milieu solvant inerte, à une température comprise entre 40°C et le reflux.
Comme solvant inerte on peut utiliser, de préférence un solvant aromatique tel que le toluène, dans un solvant dipolaire aprotique tel que le N,N-diméthylformamide ou au sein de l'agent halogénant lui-même si ce dernier est liquide. Comme agent halogénant, on peut utiliser le chlorure de thionyle, le pentachlorure, le trichlorure ou l'oxychlorure de phosphore, le chlorure d'oxalyle ou le phosgène. Pratiquement, on coule la dione de formule V dans une suspension ou une solution de l'agent halogénant (1 à 2 équivalent) dans le solvant choisi à une température comprise entre O°C et le reflux du mélangeréactionnel. Lorsque l'addition est terminée, on chauffe le mélange réactionnel à une température comprise entre 20°C et le reflux du mélange réactionnel pour terminer la réaction. Le mélange est hydrolysé à l'eau et le produit réactionnel est isolé par les méthodes classiques et éventuellement purifié par recristallisation.
Les diones de formule V sont des produits nouveaux, qui font également partie de l'invention et peuvent être obtenus par réaction d'un ester fluoroacétique avec une acétophénone dans un solvant anhydre, en présence d'une base non aqueuse, selon le schéma: 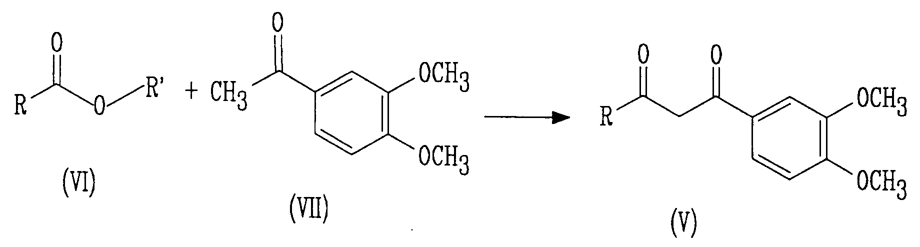
dans lequel R est un alkyle de 1 à 2 atomes de carbone, substitué par 2 à 5 atomes de fluor et R' est un alkyle de 1 à 4 atomes de carbone.
Selon une première variante la condensation de l'haloènone I avec l'acétoamide II est effectuée en présence d'une quantité stoechiométrique de base avec obtention du composé Z1, que l'on soumet successivement à:
a) une réduction, pour donner le composé III selon le schéma: 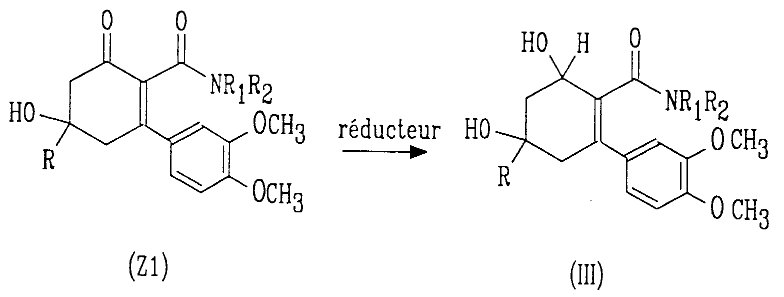 dans lequel R, R1, et R2 ont les mêmes significations que pour le composé Z1,
   en présence d'un réducteur, en milieu solvant, et que ensuite
b) le composé m est soumis à une aromatisation selon le schéma:

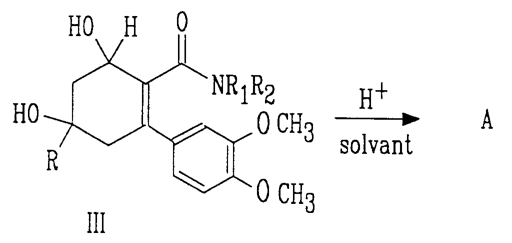

par chauffage à une température comprise entre 50 et 120°C, en milieu solvant anhydre, en présence de traces d'acide fort.
La réaction de réduction s'effectue en présence d'un réducteur, en milieu solvant. Le solvant peut être par exemple un alcool aliphatique tel que l'éthanol ou le méthanol, un éther aliphatique tel que le tétrahydrofurane ou un mélange de plusieurs de ces solvants. Comme réducteur, on utilise de préférence un hydrure mixte de métal alcalin comme par exemple le borohydrure de sodium ou de potassium. Pratiquement, l'hydroxycyclohexènone est mise en suspension dans le solvant choisi puis traitée à une température comprise entre 0°C et la température ambiante par une solution aqueuse ou alcoolique du réducteur choisi. Lorsque toute l'hydroxycyclohexènone a réagi, on évapore sous pression réduite le solvant, traite par l'eau, extrait à l'aide d'un solvant organique, sèche puis évapore pour obtenir un résidu constitué d'un mélange de deux diastéréoisomères qu'il est inutile de séparer pour l'étape suivante. Ces produits cristallisent assez difficilement.
La réaction d'aromatisation s'effectue par chauffage du diol précédent au sein d'un solvant anhydre qui peut être un hydrocarbure aromatique tel que le toluène en présence de traces d'acides minéraux ou organiques forts tels que l'acide sulfurique ou l'acide p-toluènesulfonique et en entrainant de façon continue l'eau formée. Lorsque la réaction est terminée, le solvant est évaporé et le produit est isolé par les méthodes classiques et éventuellement purifié par recristallisation.
Selon une seconde variante, la condensation de l'haloènone I avec l'acétoamide II est effectuée en présence d'une quantité de base double de la stoechiométrie avec obtention du composé Z2, que l'on soumet successivement à:
a) une réaction d'activation, avec un agent activant VI, pour donner un produit nouveau IV, qui fait partie de l'invention, selon le schéma: 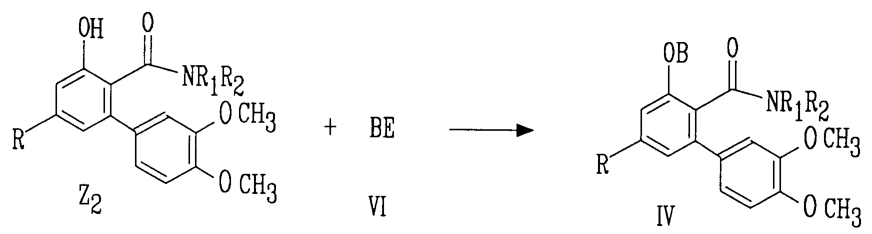 dans lequel R, R1, et R2 ont la même signification que dans Z2 et l'agent activant BE, dans lequel B est la partie labile se fixant sur l'hydroxyle et E un reste, est choisi dans le groupe comprenant le chlorure de cyanuryle, le 5-chloro-2-phényl tétrazole, les chlorures et anhydrides d'alkylsulfonyles éventuellement halogénés, le chlorure de saccharine et l'anhydride sulfurique.
b) une réduction du composé IV en composé A, par l'hydrogène gazeux en présence d'un catalyseur usuel d'hydrogénation, selon le schéma:

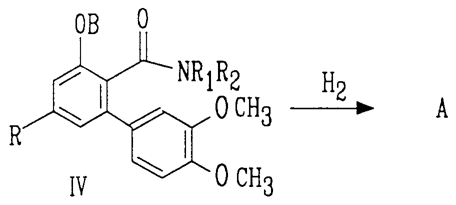

Comme catalyseur on peut citer à titre d'exemple, le palladium sur charbon ou le nickel de Raney.
L'invention concerne également, à titre de composés nouveaux les composés I, Z1, Z2, III, IV et V utilisables comme intermédiaires pour la fabrication du composé A.

L'invention concerne également un procédé de préparation du composé Z2, caractérisé en ce qu'on traite Z1 par une base en milieu solvant organique.

Les exemples suivants sont donnés pour illustrer, sans le limiter, le procédé selon l'invention.

### Exemple 1 : 1-(3,4-diméthoxyphényl)-4,4,4-trifluorobutan-1,3-dione.

Dans un ballon de 10 litres, on introduit 216 g (4 mol) de méthylate de sodium anhydre et 3,5 l de toluène puis, sous vive agitation, on coule 705 g (5 mol) de trifluoroacétate d'éthyle; la température s'élève lentement à 30°C. Après refroidissement dans un bain de glace, on ajoute goutte à goutte au mélange réactionnel, une solution de 720 g (4 mol) de 3,4-diméthoxyacétophénone dans 0,5 l de toluène; il se forme peu à peu un précipité blanc. Le mélange est ensuite abandonné sous agitation pendant une nuit puis chauffé à 50°C pendant trois heures. Après refroidissement, on ajoute 2 l d'eau et 330 ml d'acide chlorhydrique concentré, décante la phase organique, séche sur sulfate de magnésium, filtre et évapore pour obtenir 1060 g (96 %) d'un solide jaune fondant à 87 - 89 °C.

De la même façon, on a obtenu:
la 1-(3,4-diméthoxyphényl)-4,4,5,5,5-pentafluoropentan-1,3-dione fondant à 72°C.

### Exemple 2 : 4-chloro-4-(3,4-diméthoxyphényl)-1,1,1-trifluorobut-3-ène-2-one(cis).

Dans un ballon de 20 litres, on introduit 1187 g (5,7 mol) de pentachlorure de phosphore et 5l de toluène anhydre, refroidit puis coule rapidement 1060 g (3,8 mol) de 1-(3,4-diméthoxyphényl)-4,4,4-trifluorobutan-1,3-dione. en solution dans 4 l de toluène. Le mélange réactionnel est rapidement porté à 60°C puis maintenu à cette température pendant 2 heures. Le mélange réactionnel est versé sur 10 l d'eau et 5 Kg de glace, extrait avec 5 l d'acétate d'éthyle, lavé avec de la soude diluée séché sur sulfate de sodium, filtré et évaporé pour fournir 1132 g d'un solide de couleur brun foncée. Le produit brut est recristallisé dans 3 l d'éthanol pour fournir 741 g (rendement: 67%) d'un solide jaune citron fondant à 98 - 100 °C.

De la même façon, nous avons obtenu:
5-chloro-5-(3,4-diméthoxyphényl)-1,1,1 ,2,2-pentafluoropent-4-ène-3-one (Z) fondant 67 C.

### Exemple 3 : N-éthyl,N-méthyl acétoacétamide

Dans un autoclave en inox de 1l, on introduit 158 g (1 mol) d'acétoacétate de ter-butyle 61 g de N-éthyl,N-méthylamine (1,05 mol) puis chauffe à 130°C pendant six heures; la pression ne dépasse pas 10 bars. Le contenu de l'autoclave est transféré dans un ballon et le ter-butanol formé est évaporé sous pression réduite pour fournir 140 g (98%) de N-éthyl,N-méthyl acétoacétamide suffisament pur pour être engagé dans la réaction suivante.
Les autres acétoacétamides sont disponibles dans le commerce.

### Exemple 4 : N.N-diéthyl-1-carboxamide-4-trifluorométhyl-4-hydroxy-6-(3.4-diméthoxy phényl) cyclohex-6-ène-2-one.

Dans un ballon de 10 litres, on introduit 2800 g (9,5 mol) de 4-chloro-4-(3,4-diméthoxyphényl)-1,1,1-trifluorobut-3-ène-2-one et 4,5 l d'éthanol anhydre, puis coule rapidement 1790 g (11,4 mol) d'acétoacétamide de N,N-diéthyle. On ajoute alors en cinq fois à deux heures d'intervalle, à température ambiante, un total de 1670 g (5,3 mol) de baryte hydratée Ba(OH)₂, 8H₂O) tout en agitant. Après une nuit sous agitation, le mélange réactionnel est lentement porté au reflux puis maintenu à cette température pendant 6 heures. Le mélange réactionnel est filtré sur de la terre de diatomées, lavé par 2,5 l d'éthanol et 2,5 l d'acétate d'éthyle froid. Le solide blanc ainsi obtenu est séché à 50 C sous pression réduite pour fournir 2548 g ( 64,6%) d'un solide blanc fondant à 174°C.

De la même façon, on obtient les dérivés suivants:
N-éthyl,N-méthyl-1-carboxamide-4-trifluorométhyl-4-hydroxy-6-(3,4-diméthoxyphényl)-cyclohex-6-ène-2-one fondant à 163 -165°C.
1-(4-morpholino)carbonyl-4-trifluorométhyl-4-hydroxy-6-(3,4-diméthoxy-phényl)-cyclohex-6-ène-2-one fondant à 183°C.
N-éthyl,N-méthyl-1-carboxamide-4-(pentafluoro-1,1,1,2,2-éthyl)-4-hydroxy-6-(3,4-diméthoxyphényl)-cyclohex-6-ène-2-one fondant à 163 °C.
N,N-diéthyl-1-carboxamide-4-(pentafluoro-1,1,1,2,2-éthyl)-4-hydroxy-6-(3,4-diméthoxyphényl)-cyclohex-6-ène-2-one fondant à 174 - 176 °C.

### Exemple 5 : N,N-diéthyl-1-carboxamide-4-trifluorométhyl-6-(3,4-diméthoxyphényl) cyclohex-6-ène-2,4-diol.

Dans un ballon de 20 litres, on introduit 1388 g (3.31mol) de N,N-diéthyl-1-carboxamide-4-trifluorométhyl-4-hydroxy-6-(3,4-diméthoxyphényl) cyclohex-6-ène-2-one et 12 l de méthanol anhydre. Puis tout en agitant, on coule rapidement une solution de 146 g (3,82 mol) de borohydrure de sodium dans 1500ml d'eau glacée dans le mélange réactionnel refroidi à 0°C. Lorsque l'addition est terminée, on laisse revenir le mélange à température ambiante et abandonne sous agitation pendant trois heures. Le milieu est ensuite filtré sur de la terre de diatomées, puis évaporé sous pression réduite. Le résidu est repris par l'eau, extrait au dichlorométhane, lavé à l'eau puis évaporé sous pression réduite pour fournir 1322 g ( rendement: 95,2 %) d'une huile légèrement colorée cristallisant lentement. Le produit ainsi obtenu est utilisé tel quel pour l'étape suivante.

De la même façon, on obtient les dérivés suivants:
N-éthyl,N-méthyl-1-carboxamide-4-trifluorométhyl-6-(3,4-diméthoxyphényl) cyclohex-6-ène-2,4-diol.
1-(4-morpholino)carbonyl-4-trifluorométhyl-6-(3,4-diméthoxyphényl) cyclohex-6-ène-2,4-diol.
N-éthyl,N-méthyl-1-carboxamide-4-(pentafluoro-1,1,1,2,2-éthyl)-6-(3,4-diméthoxyphényl) cyclohex-6-ène-2,4-diol.
N,N-diéthyl-1-carboxamide-4-(pentafluoro-1,1,1,2,2-éthyl)-6-(3,4-diméthoxyphényl) cyclohex-6-ène-2,4-diol.

### Exemple 6: N.N-diéthyl-4-trifluorométhyl-2-(3,4-diméthoxyphényl)benzamide.

Dans un ballon de 20 litres, on introduit 2429 g (5,82 mol) de N,N-diéthyl-1-carboxamide-4-trifluorométhyl-6-(3,4-diméthoxyphényl) cyclohex-6-ène-2,4 diol, 13 l de toluène anhydre et 118 g d'acide p-toluènesulfonique monohydraté. Le mélange réactionnel est porté au reflux tout en éliminant de façon continue l'eau formée avec un dispositif de Dean et Starck . Lorsqu'il ne se forme plus d'eau, la réaction est terminée; le mélange réactionnel est alors filtré sur de la terre de diatomées et évaporé sous pression réduite. Le résidu est repris par l'eau, extrait au dichlorométhane, lavé à l'eau puis évaporé sous pression réduite pour fournir 2175 g (rendement: 98 %) d'une huile légèrement colorée qui cristallise lentement. Le produit ainsi obtenu peut être purifié par recristallisation dans un mélange d'éther isopropylique et de pentane, pour donner un solide blanc fondant à 108 -110°C.

De la même façon, on a obtenu les dérivés suivants:
N-éthyl,N-méthyl-4-trifluorométhyl-2-(3,4-diméthoxyphényl)benzamide fondant à 103 - 105 °C
1-(4-morpholino)carbonyl-4-trifluorométhyl-2-(3,4-diméthoxyphényl) benzène fondant à 183 - 185 °C
N-éthyl,N-méthyl-4-(pentafluoro-1,1,1 ,2,2-éthyl)-2-(3,4-diméthoxy phényl) benzamide fondant à 104 - 106°C.
N,N-diéthyl-4-(pentafluoro-1,1,1,2,2-éthyl)-2-(3,4-diméthoxyphényl) benzamide fondant à 94°C.

### Exemple 7 : N.N-diéthyl-4-trifluorométhyl-2-hydroxy-6-(3,4-diméthoxy phényl) benzamide

Dans un ballon de 150 ml, on introduit 28,0 g (0,095 mol) de 4-chloro-4-(3,4-diméthoxyphényl)-1,1,1-trifluorobut-3-ène-2-one et 100 ml d'éthanol anhydre, puis coule rapidement 18,0 g (0,115 mol) d'acétoacétamide de diéthyle. On ajoute alors en cinq fois à deux heures d'intervalle un total de 33,4 g (0,11 mol) de baryte hydratée Ba(OH)₂, 8H₂O) tout en agitant. Après une nuit sous agitation, le mélange réactionnel est lentement porté au reflux puis maintenu à cette température pendant 12 heures. Le mélange réactionnel est acidifié avec de l'acide chlorhydrique, extrait par 3 fois 250 ml d'éther éthylique et rincé à l'eau. La phase organique est ensuite extraite par 3 fois 100 ml de soude 3N, la phase organique est rejetée, la phase aqueuse est extraite par 2 fois 200 ml d'éther diéthylique, filtrée sur Supercel puis acidifiée avec de l'acide chlorhydrique; le produit qui se forme est extrait par 3 fois 250 ml de dichlorométhane pour fournir 32g (85 %) d'un solide beige fondant à 161°C.

De la même façon, on a obtenu les dérivés suivants:
N-éthyl,N-méthyl-4-trifluorométhyl-2-hydroxy-6-(3,4-diméthoxyphényl) benzamide fondant à 114°C.
1-(4-morpholino)carbonyl-4-trifluorométhyl-2-hydroxy-6-(3,4-dim éthoxyphényl) benzène fondant à 231°C
N,N-diméthyl-4-trifluorométhyl-2-hydroxy-6-(3,4-diméthoxyphényl) benzamide fondant à 129°C.

### Exemple 8 : N,N-diéthyl-4-trifluorométhyl-2-trifluorométhylsulfonato-6-(3,4-diméthoxy phényl) benzamide.

A un mélange de 1,0 g (2,6 mmol) de N,N-diéthyl-4-trifluorométhyl-2-hydroxy-6-(3,4-diméthoxy phényl) benzamide précédent, et de 1,5 ml de triéthylamine (11,6 ml) dans 5 ml de dichlorométhane à 0°C, on ajoute goutte à goutte 0,5 ml (3 mmol) d'anhydride trifluorométhanesulfonique. Après quelques minutes: la réaction est terminée; on extrait à l'éther diéthylique, lave à l'eau, évapore le solvant et cristallise le produit dans l'hexane pour founir 0,8 g (57% ) de produit fondant à 122°C.

### Exemple 9 : N,N-diéthyl-4-trifluorométhyl-2-(3,4-diméthoxy phényl) benzamide.

Dans un ballon sous argon, on met 0,73 g (1,4 mmol) du triflate précédemment préparé, 1,4 ml de n-tributylamine, 87 mg (0,21 mmol) de DPPP, 52 mg (0,074 mmol) de Pd(Ph₃P)₂Cl₂, 4,2 ml de DMF et 0,14 ml (3,7 mmol) d'acide formique. On chauffe à 110°C pendant une heure. Après refroidissement, on extrait à l'éther, lave à l'eau pour obtenir 0,7 g d'un mélange de produit attendu et de N,N-diéthyl-4-trifluorométhyl-2-hydroxy-6-(3,4-diméthoxy phényl) benzamide. Le produit voulu est extrait sélectivement par l'éther en milieu basique. Après lavage par l'eau et évaporation du solvant sous vide, on obtient 0,34 g (66%) de produit attendu fondant à 108 - 110°C.

## Revendications

**1)** Procédé de préparation de composés de procédé pour le préparation de dérivés phénylbenzamides de formule A, 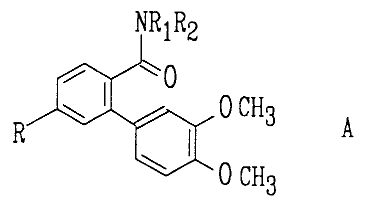 dans laquelle R est un alkyle de 1 à 2 atomes de carbone, substitué par 2 à 5 atomes de fluor, R1 et R2, identiques ou différents sont chacun un méthyle ou un éthyle ou peuvent former ensemble un groupe morpholino, caractérisé en ce qu'on effectue une condensation d'une haloénone de formule I, dans laquelle R a la même signification que ci-dessus, avec un acétoamide de formule II, dans laquelle R1 et R2 ont les même significations que ci-dessus selon le schéma: 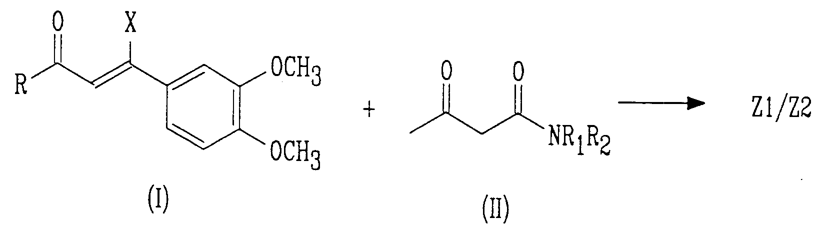 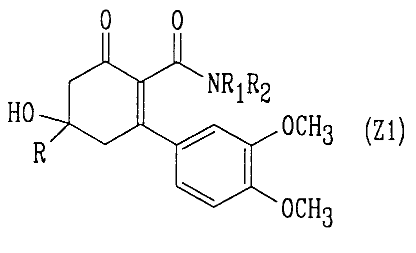 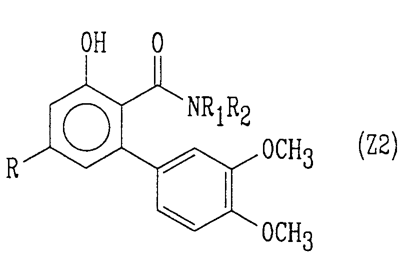 en présence d'un solvant organique et d'un à deux équivalents de base, et qu'on soumet le produit résultant à une transformation en deux étapes dont une de réduction.

**2)** Procédé selon la revendication 1, caractérisé en ce que la condensation est effectuée en présence d'une quantité stoechiométrique de base avec obtention du composé Z1, que l'on soumet successivement a:
a) une réduction, pour donner le composé III selon le schéma: 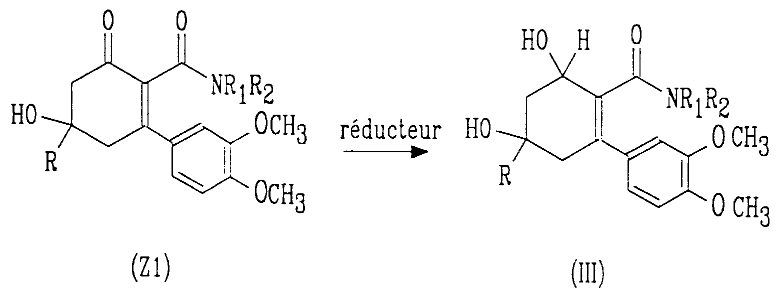 dans lequel R, R1 et R2 ont la même signification que dans la formule A,
en présence d'un réducteur, en milieu solvant, et que
b) le composé III est soumis à une aromatisation selon le schéma: 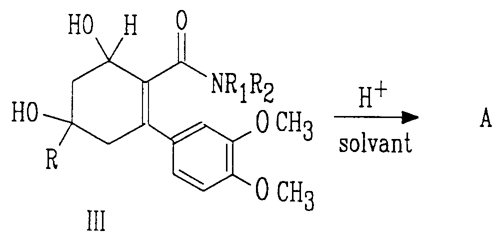
par chauffage à une température comprise entre 50 et 120°C, en milieu solvant anhydre, en présence de traces d'acide fort.

**3)** Procédé selon la revendication 2, caractérisé en ce que la réduction s'effectue en utilisant comme réducteur un hydrure mixte de métal alcalin.

**4)** Procédé selon la revendication 2, caractérisé en ce que la réduction s'effectue en milieu solvant choisi dans le groupe comprenant un alcool aliphatique, un éther aliphatique ou un mélange de plusieurs de ces solvants.

**5)** Procédé selon la revendication 2 caractérisé en ce que pour l'aromatisation, on opère dans un solvant aromatique.

**6)** Procédé selon la revendication 2 caractérisé en ce que pour l'aromatisation, on opère en présence de traces d'acide sulfurique ou d'acide para-toluènesulfonique.

**7)** Procédé selon la revendication 1 caractérisé en ce que la condensation est effectuée en présence d'une quantité de deux équivalents de base avec obtention du composé Z2, que l'on soumet successivement à:
a) une réaction d'activation en un produit IV avec un agent activant VI, selon le schéma: 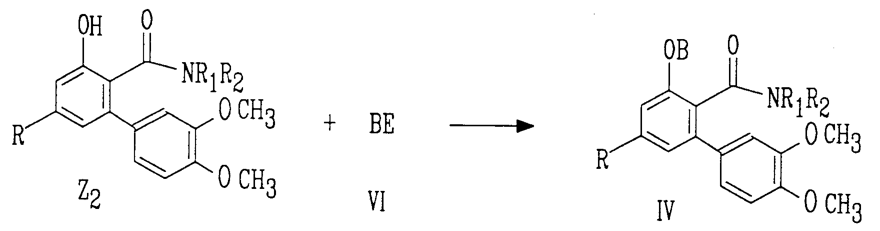 dans lequel R, R1 et R2 ont les mêmes signification que précédemment et B est la partie labile de l'agent activant,
puis
b) une réduction du composé IV en composé A, par l'hydrogène gazeux en présence d'un catalyseur usuel d'hydrogénation, selon le schéma:

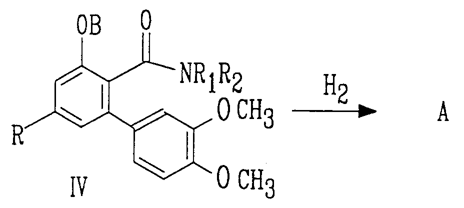

**8)** Procédé selon la revendication 7, caractérisé en ce que l'agent activant VI est choisi dans le groupe comprenant le chlorure de cyanuryle, le 5-chloro-2-phényl tétrazole, les chlorures et anhydrides d'alkylsulfonyles éventuellement halogénés, le chlorure de saccharine et l'anhydride sulfurique.

**9)**Dérivé haloénone de formule I: 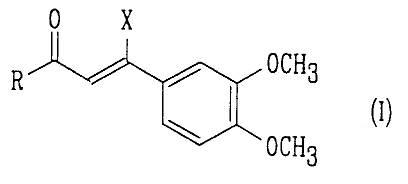 dans laquelle R est un alkyle de 1 à 2 atomes de carbone, substitué par un 2 à 5 atomes de fluor, et X est un atome d'halogène.

**10)** Procédé de fabrication d'une haloénone selon la revendication 9, caractérisé en qu'on effectue une halogénation de la dione correspondante, de formule V, selon le schéma: 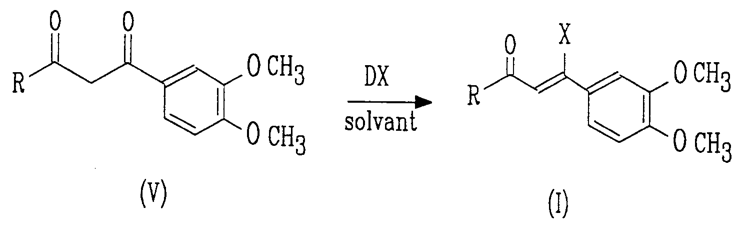 dans lequel R est un alkyle de 1 à 2 atomes de carbone, substitué par un 2 à 5 atomes de fluor, et DX est un agent halogénant dont la partie labile est X, en milieu solvant.

**11)** Procédé selon la revendication 10, caractérisé en ce que la température est comprise entre 40°C et la température de reflux du milieu réactionnel.

**12)**Procédé selon la revendication 10, caractérisé en ce que l'agent halogénation est un agent de chloration choisi dans le groupe comprenant le chlorure de thionyle, le tri ou oxy ou pentachlorure de phosphore, le chlorure d'oxalyle ou le phosgène.

**13)** Dérivé dione de formule V: 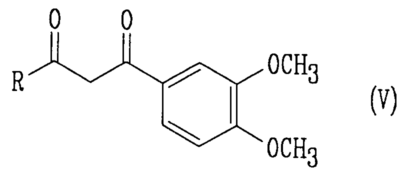 dans laquelle R est un alkyle de 1 à 2 atomes de carbone, substitué par un 2 à 5 atomes de fluor, R ne pouvant pas être un trifluorométhyle.

**14)** Procédé de fabrication d'une dione selon la revendication 13, caractérisé en ce qu'on fait réagir un ester fluoroacétique avec une acétophénone dans un solvant anhydre, en présence d'une base non aqueuse, selon le schéma: 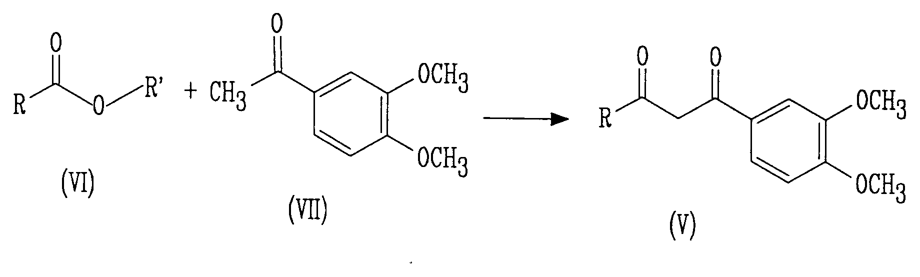 dans lequel R est un alkyle de 1 à 2 atomes de carbone, substitué par 2 à 5 atomes de fluor, R ne pouvant pas être un trifluorométhyle et R' est un alkyle de 1 à 4 atomes de carbone.

**15)** Dérivé de formule Z1, 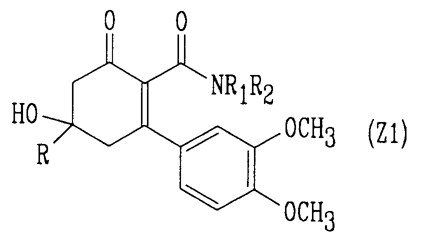 dans laquelle R est un alkyle de 1 à 2 atomes de carbone, substitué par un 2 à 5 atomes de fluor, R1 et R2, identiques ou différents sont chacun un méthyl ou un éthyl ou peuvent former ensemble un groupe morpholino.

**16)** Dérivé de formule Z2, 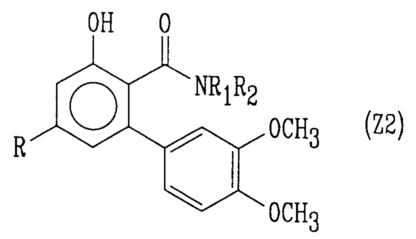 dans laquelle R est un alkyle de 1 à 2 atomes de carbone, substitué par un 2 à 5 atomes de fluor, R1 et R2, identiques ou différents sont chacun un méthyl ou un éthyl ou peuvent former ensemble un groupe morpholino.

**17)** Procédé de fabrication du dérivé Z2 selon la revendication 16, caractérisé en ce qu'on traite le dérivé Z1 par une base en milieu solvant organique, selon le schéma: 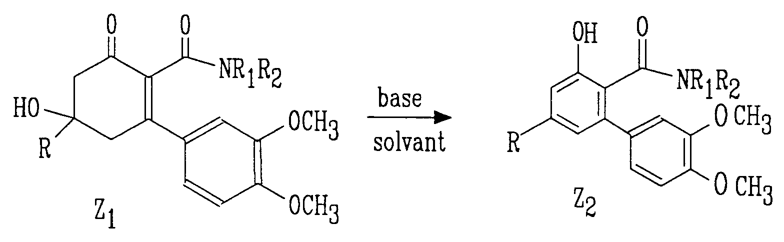 en milieu solvant inerte, à une température comprise entre 40°C et le reflux du milieu réactionnel.

**18)** Dérivé de formule III: 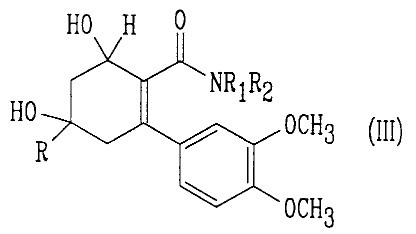 dans laquelle R est un alkyle de 1 à 2 atomes de carbone, substitué par un 2 à 5 atomes de fluor, R1 et R2, identiques ou différents sont chacun un méthyl ou un éthyl ou peuvent former ensemble un groupe morpholino.

**19)** Dérivé de formule IV 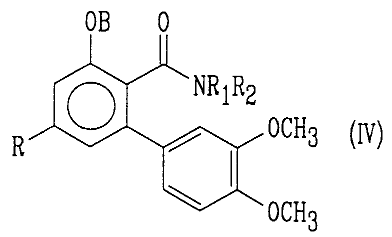 dans laquelle R est un alkyle de 1 à 2 atomes de carbone, substitué par un 2 à 5 atomes de fluor, R1 et R2, identiques ou différents sont chacun un méthyle ou un éthyle ou peuvent former ensemble un groupe morpholino.
